# FASCICULE DE BREVET EUROPEEN

(11) **EP 1 448 208 B1**
(45) Date de publication et mention de la délivrance du brevet: **03.01.2007**
(21) Numéro de dépôt: 02795379.3
(22) Date de dépôt: 18.11.2002
(51) Int. Cl.: A61K 31/58, A61K 8/63, A61P 17/00

(54) **COMPOSITION A BASE D'ESTER DE DIOSGENINE APPLICABLE PAR VOIE TOPIQUE**
TOPISCH ANWENDBARE ZUSAMMENSTELLUNG AUF BASIS VON DIOSGENINESTERN
COMPOSITION BASED ON DIOSGENIN ESTER FOR TOPICAL USE

(30) Priorité: 30.11.2001 FR 0115536
(43) Date de publication de la demande: 25.08.2004
(73) Titulaire: Laboratoire de Dermo-Cosmologie, 75006 Paris (FR)
(72) Inventeur: EYMARD, Michèle, F-92190 Meudon (FR)
(74) Mandataire: L'Helgoualch, Jean
(86) Numéro de dépôt international: PCT/FR2002/003939
(87) Numéro de publication internationale: WO 2003/045399

(56) Documents cités:
- EP-A- 0 162 330
- FR-A- 2 786 097
- FR-M- 2 367
- GB-A- 1 349 050
- DATABASE WPI Section Ch, Week 199241 Derwent Publications Ltd., London, GB; Class B01, AN 1992-334768 XP002201050 & JP 04 230696 A (SANWA SHOYAKU KK), 19 août 1992 (1992-08-19)

## Description

La présente invention concerne une composition utile en thérapeutique humaine ou animale et en cosmétologie, et plus particulièrement une composition à action locale applicable par voie topique comprenant un ester de diosgénine.

La diosgénine, analogue structurel du cholestérol, est essentiellement utilisée en thérapeutique en raison dé ses propriétés anti-inflammatoires et de son action de compensation des effets délétères des oestrogènes, plus particulièrement en période de périménopause et de ménopause. Elle est aussi utilisée en cosmétique et en dermatologie pour ses propriétés antiseptiques cutanées.

La diosgénine peut être extraite de plantes du genre dioscorea, en particulier dioscorea opposita, qui est une plante utilisée en médecine traditionnelle chinoise. L'extraction peut être faite par les techniques usuelles, par exemple par extraction éthanolique, à partir de toute partie de la plante, et de préférence le rhizome, préalablement séché et broyé. Une méthode de culture de plantes de la famille des dioscorea, en particulier dioscorea floriburida et dioscorea composita, permettant d'améliorer la teneur en diosgénine est décrite dans le brevet US-A-3.918.200.

Le brevet FR 2.367 M décrit l'utilisation de la diosgénine en tant que médicament présentant une activité antiphlogistique et anti-inflammatoire utile pour le traitement d'affections rhumatismale et vasculaires. Le brevet FR-A-2.786.097 décrit des compositions cosmétiques amincissantes comprenant environ 5% d'extraits de dioscorea opposita, ayant pour effet d'agir sur le métabolisme lipidique de l'adipocyte en limitant l'accumulation des graisses dans la cellule. Le brevet US-A-5.804.211 décrit une composition sous forme de lotion nasale à base végétale destinée à limiter ou supprimer le ronflement, et contenant un extrait de dioscorea, du gingembre, de la camomille et un sel de potassium ou de sodium. L'extrait de dioscorea est de préférence dioscorea villosa, utilisé à raison de 0,01 à 0,4% en poids par rapport au poids total de la composition.

L'utilisation de la diosgénine dans des compositions thérapeutiques a aussi été décrite, par exemple dans le brevet EP-A-873.125 relatif à une composition associant la diosgénine à des vitamines, telles que vitamine K et vitamine D, administrable par voie orale ou transdermique, pour le traitement de l'ostéoporose.

On sait aussi que la diosgénine, saponine stéroïdienne, est un précurseur de divers stéroïdes, et notamment de la DHEA qui est une hormone importante de l'organisme humain ou animal, produite par les glandes surrénales, et qui est transformée en oestrogène et testostérone. Cependant, la diosgénine ne se transforme pas directement en progestérone, mais en régulateur hormonal. Rien dans la littérature scientifique ne permet de supposer que la diosgénine, en particulier lorsqu'elle est administrée par voie topique, pourrait avoir la même activité que la DHEA, notamment parce que les enzymes susceptibles de la transformer dans l'organisme n'ont jamais été mises en évidence. Par contre, on sait préparer de la DHEA en laboratoire à partir de diosgénine.

Les études cliniques réalisées par la demanderesse ont permis de montrer que des dérivés de diosgénine, en particulier des esters de diosgénine, possèdent des propriétés utiles en dermatologie et en cosmétique, et notamment une action sur le système musculo-aponévrotique superficiel lorsqu'ils sont administrés par voie topique, externe ou transdermique.

La présente invention a donc pour objet une composition utilisable en thérapeutique et en cosmétologie par voie topique comprenant un ester de diosgénine.

L'invention a encore pour objet une composition cosmétique et/ou dermatologique à teneur déterminée en ester de diosgénine.

L'invention a enfin pour objet l'utilisation d'un ester de diosgénine pour la fabrication d'un médicament dermatologique agissant par action similaire à celle de la progestérone et des oestrogènes sur le système musculo-aponévrotique superficiel ou sur les récepteurs cellulaires des oestrogènes et de la progestérone, pour le traitement des affections de la peau, ainsi qu'un procédé de traitement cosmétique de la peau.

La présente invention a aussi pour objet une composition possédant un effet adaptagène intracellulaire ou extracellulaire au niveau des récepteurs des cellules de l'épiderme, du derme du système musculo-aponévrotique superficiel, et de l'hypoderme, et permettant son application en dermatologie et en cosmétique, notamment pour la régénération et la restructuration cutanées, et d'une manière générale pour lutter contre les signes du vieillissement cutané.

La composition cosmétique et/ou dermatologique de l'invention se distingue en ce qu'elle contient un ester de diosgénine à chaîne ester alkylée linéaire ou ramifiée de 1 à 30 atomes de carbone.

Le reste ester porté par la diosgénine peut provenir d'un acide comportant de préférence 1 à 30 atomes de carbone, et par exemple un acide gras, un amino-acide ou encore un alpha-hydroxy-acide. L'acide gras peut être par exemple l'acide stéarique, oléique ou linoléique; l'amino-acide est choisi de préférence parmi la leucine, la proline, et la sérine ; l'alpha-hydroxy acide est choisi de préférence parmi les acides glycolique, lactique, mandélique et citrique. Il peut être tout particulièrement avantageux d'utiliser le lactate ou le glycolate de diosgénine.

Suivant une variante conforme à la présente invention, un alpha-hydroxy acide tel que l'acide glycolique, l'acide lactique, l'acide thiolactique ou l'acide thioglycolique ou un de leurs sels, est utilisé conjointement avec l'ester de diosgénine.

Il est particulièrement avantageux, conformément à la présente invention, d'utiliser l'ester de diosgénine en combinaison avec un mucopolysaccharide, en particulier un mucopolysaccharide hydrolysé.

Les études effectuées ont montré que les esters de diosgénine suivant l'invention, appliqués par voie topique, par exemple sous forme de gel, crème ou lotion, pénètrent mieux dans l'épiderme et présentent une biodisponibilité améliorée par rapport à la diosgénine pure.

L'ester de diosgénine peut être préparé suivant les techniques usuelles en faisant réagir la diosgénine avec l'acide correspondant, dans des conditions opératoires appropriées. La diosgénine peut être elle-même obtenue à partir d'extrait de plantes de la famille des dioscoreacées.

L'extrait de dioscorea utilisé dans la présente invention pour la préparation d'un ester de diosgénine peut être tout extrait de dioscorea de la famille des dioscoreacées, et par exemple dioscorea opposita, dioscorea composita, dioscorea floriburida, dioscorea villosa, dioscorea hypoglauca, yam, wild yam, et de préférence dioscorea opposita. Il est obtenu sous forme d'extrait de toute la plante ou, de préférence, du rhizome, comme extrait sec ou huile essentielle. Comme indiqué ci-dessus, la préparation de l'ester de diosgénine peut être réalisée par tout procédé adapté, par action d'un acide approprié sur la diosgénine ou sur l'extrait de dioscorea ayant une teneur satisfaisant en diosgénine.

Comme indiqué ci-dessus, les compositions suivant la présente invention contiennent une teneur déterminée en ester de diosgénine, qui peut être relativement élevée par rapport aux doses usuelles de principes actifs dermatologiques, et être comprise entre 0,05 % et 80 % en poids par rapport au poids total de la composition et de préférence entre 0,5 % et 30 % en poids. Ces teneurs en esters peuvent être choisies en fonction du mode d'administration envisagé. Par exemple, dans le cas d'un patch pour administration transdermique, la teneur en ester de diosgénine peut être avantageusement comprise entre 15 et 30 % en poids.

L'effet adaptagène de la composition de l'invention résulte de la teneur élevée en ester de diosgénine procurant une action efficace se traduisant par une activation de l'effet similaire à celui de la progestérone dans l'organisme, et une modulation des récepteurs cellulaires des oestrogènes et de la progestérone.

En particulier, la composition de l'invention exerce ses effets sur le système musculo-aponévrotique superficiel et sur tous les tissus en général, en provoquant une augmentation de la sensibilité des récepteurs membranaires ou cytoplasmiques des cellules, aux hormones circulantes. Cet effet permet donc de compenser la diminution du nombre de récepteurs par une augmentation de leur sensibilité. Le système musculo-aponévrotique superficiel est une trame fibreuse conjonctive contenant des fibroblastes sensibles aux oestrogènes circulants et des fibres musculaires sensibles à la progestérone.

Cette action est particulièrement efficace sur la peau présentant des signes de vieillissement cutané en compensant la perte hormonale par une augmentation de l'activité liée à l'augmentation de la sensibilité des récepteurs.

Cette activité peut être avantageusement mise à profit dans le traitement des signes du vieillissement cutané en entraînant une augmentation de l'épaisseur cutanée par une densification de la structure de l'épiderme, du derme et de l'hypoderme, ainsi que du derme du système musculo-aponévrotique superficiel. Les signes du vieillissement cutané sont plus particulièrement ceux qui se caractérisent par un relâchement de la structure cutanée et une diminution de l'épaisseur de la peau, et, comme indiqué ci-dessus, la composition suivant l'invention agit en compensant la perte hormonale par l'augmentation de la sensibilité des récepteurs aux oestrogènes et à la progestérone. Cette activité peut être aussi mise à profit dans les traitements dermatologiques et de chirurgie esthétique en procurant une densification et une restructuration de la peau et une augmentation de la tension musculaire, notamment du visage.

La composition de l'invention peut aussi être utilisée avantageusement pour le traitement de diverses affections de la peau, et par exemple pour le traitement des brûlures, de la cicatrisation, pour une action anti-oedémateuse dans un traitement associé au niveau par exemple de l'insuffisance veineuse ou des adiposités localisées.

L'utilisation de l'acide thiolactique ou de l'acide thioglycolique ou un de leurs sels, en combinaison avec l'ester de diosgénine suivant l'invention s'avère particulièrement efficace dans l'application au défrisage des fibres kératiniques, cils ou cheveux.

Enfin, la composition suivant l'invention est utile dans le traitement des surcharges pondérales. En effet, les oestrogènes et progestérones sont les agents hormonaux connus de l'obésité des membres du corps humain. Au niveau des adipocytes, la répartition des récepteurs α-adrénergiques et des récepteurs β-adrénergiques est d'environ 85/15. De plus, on sait que la face antéro-interne de la cuisse comprend des graisses de type I ayant une sensibilité particulière aux oestrogènes et une activité lipoprotéine lipasique élevée, tandis que la face externe de la cuisse a une sensibilité particulière à la progestérone dont on connaît l'action anti-oedémateuse. Ainsi, des troubles de la régulation hormonale peuvent occasionner une diminution, voire une disparition, des récepteurs β-adrénergiques qui favorise la lipolyse plus particulièrement dans la région pelvienne et à l'intérieur des genoux, pouvant entraîner des dysmorphies de ces régions. De plus, des troubles vasculaires peuvent favoriser encore la disparition des capteurs β. IL en résulte une accumulation de graisses sur la face interne des cuisses en particulier chez une femme hyperoestrogénique présentant des troubles circulatoires.

La composition suivant l'invention permet de lutter contre ces surcharges pondérales en rétablissant un trophisme cutané et une microcirculation normale permettant la libération de certains récepteurs β-adrénergiques. Le rétablissement de la lipolyse et de la microcirculation périnodulaire est mise en évidence par l'augmentation de l'amplitude et la modification des tracés pléthysmographiques.

Les esters de diosgénine suivant la présente invention peuvent être formulés suivant les techniques usuelles et être administrés sous forme d'émulsions, gels, masques, lotions, solutions pour pulvérisation, etc. Les émulsions peuvent être du type huile dans eau (H/E) ou eau dans huile (E/H), par exemple des gels ou des crèmes. On peut aussi recourir aux techniques d'encapsulation et par exemple à la technique d'encapsulation dans des liposomes.

L'émulsionnant utilisé pour la composition de l'invention est choisi en fonction des formulations et des conditions d'administration. Par exemple, on peut choisir l'émulsionnant parmi des polymères carboxyvinyliques à haut poids moléculaire (par exemple le Carbopol®), des polysorbates (par exemple le Tween 20® ou le Tween 60®), des esters de sorbitan et en particulier un monostéarate, un tristéarate, un monopalmitate, et un laurate de sorbitan (par exemple les émulsionnants connus sous la marque Arlacel®) On peut encore utiliser d'autres agents émulsionnants tels que divers dérivés d'acide stéarique ou palmitique, et par exemple le stéarate de PEG 100® ou de PEG 20® ou un ester en C12-C20 de PEG 8 ou PEG 9, des glycérides d'acide stéarique ou palmitique, le stéarate de glycérol, un stéarate de polyéthylène glycol, un steareth ou un ceteareth (par exemple le ceteareth 20), ou encore le polyglycéryl-2-sesquioléate, l'éther cétylique de polyoxyéthylène, ou une silicone émulsionnable.

Outre les émulsionnants mentionnés ci-dessus, les compositions peuvent contenir divers adjuvants usuels choisis parmi ceux utilisés dans les techniques de préparation de compositions cosmétiques et dermatologiques, et par exemple des conservateurs, des épaississants, des gélifiants hydrophiles ou lipophiles, des antioxydants, des agents hydratants, des tensioactifs, des parfums, des colorants, des pigments, des charges et divers additifs destinés à améliorer les propriétés physiques de la composition. Il peut aussi être avantageux d'incorporer dans la composition de l'invention, des écrans ou filtres solaires actifs dans l'UVA et/ou l'UVB, choisis en fonction du degré de protection recherché.

Les esters de diosgénine suivant la présente invention peuvent être utilisés pour la fabrication d'un médicament dermatologique agissant par action sur le système musculo-aponévrotique superficiel au niveau des récepteurs cellulaires, pour le traitement de diverses affections de la peau, ainsi que dans un procédé de traitement cosmétique de la peau consistant à appliquer sur la zone à traiter, une quantité efficace d'ester de diosgénine, isolément ou en combinaison avec des excipients et supports acceptables.

Les exemples suivants illustrent l'invention plus en détail sans en limiter la portée.

### Exemple 1

En utilisant les techniques usuelles de formulations dermatologiques, on prépare un gel exfoliant ayant la composition suivante, indiquée en parties en poids :

| | | |
|---|---|---|
| lactate de diosgénine | | 1,0 |
| acide glycolique | | 70,0 |
| agent hydratant | | 5,0 |
| pyrrolidone carboxylate de lysine | | 4,0 |
| éthanol | | 5,0 |
| hydroxyde d'ammonium | | 5,0 |
| conservateur | | 3,0 |
| parfum | | 0, 5 |
| eau | q.s.p. | 100,0 |

Ce gel exfoliant peut être utilisé par application sur la peau, une fois par semaine. Les essais effectués ont démontré un effet antirides et restructurant manifeste dès la fin de la deuxième semaine d'application sur les zones présentant les signes de vieillissement cutané constitués par des rides, un affinement de la peau et un relâchement cutané. On observe de façon remarquable un effet de tension de la structure cutanée dès la première semaine de traitement par l'action au niveau du système musculo aponévrotique superficiel.

### Exemple 2

On prépare une crème amincissante et anti-oedémateuse ayant la composition pondérale indiquée ci-après.

| | | |
|---|---|---|
| glycolate de diosgénine | | 5,0 |
| mucopolysaccharides hydrolysés | | 2,0 |
| éthanol | | 5,0 |
| agent hydratant | | 5,0 |
| C₁₂₋₂₀ PEG-8 ester | | 7,0 |
| octanoate de cétéaryle / palmitoyle | | 7,0 |
| amino-acides de soie | | 2,0 |
| extrait de ginko biloba | | 1,0 |
| troxérutine | | 1,0 |
| conservateur | | 0,2 |
| parfum | | 0,1 |
| eau | q.s.p. | 100,0 |

Cette crème a été appliquée une à deux fois par jour sur les jambes de patientes âgées de 30 à 50 ans, au niveau des cuisses et des genoux.

L'action anti-oedémateuse est visible dès le deuxième jour du traitement. Le drainage tissulaire est particulièrement appréciable après les lipoplasties des cuisses et des genoux, évitant les fibroses rétractiles. De plus, l'action au niveau du système musculo-aponévrotique superficiel est remarquable par la tension très rapide du tissu, ce qui permet d'appliquer ce traitement aux adiposités localisées. L'action prolongée entraîne une diminution de la surcharge graisseuse grâce à l'effet adaptagène qui réactive les récepteurs sensibles à la progestérone.

### Exemple 3

On prépare une composition suivant la présente invention, favorisant la repousse des cheveux, ayant la composition pondérale suivante.

| | | |
|---|---|---|
| glycolate de diosgénine | | 20,0 |
| mucopolysaccharides hydrolysés | | 0,5 |
| C₁₂₋₂₀ PEG-8 ester | | 8,0 |
| octanoate de cétéaryle | | 2,0 |
| propylène glycol dicaprylate dicaprate | | 3,0 |
| aescine | | 0,5 |
| extrait de ginko biloba | | 1,0 |
| conservateur | | 0,2 |
| eau | q.s.p. | 100,0 |

## Revendications

1. Composition utile en thérapeutique et en cosmétologie pour application par voie topique, **caractérisée en ce qu'**elle comprend un ester de diosgénine à chaîne ester alkylée linéaire ou ramifiée de 1 à 30 atomes de carbone.

2. Composition selon la revendication 1, **caractérisée en ce que** l'ester de diosgénine est choisi parmi le lactate et le glycolate de diosgénine.

3. Composition selon l'une quelconque des revendications 1 et 2, **caractérisée en ce que** la teneur en ester de diosgénine est comprise entre 0,05 % et 80 % en poids par rapport au poids total de la composition.

4. Composition selon la revendication 1, **caractérisée en ce qu'**elle comprend en outre un alpha-hydroxy acide ou un sel d'alpha-hydroxy acide.

5. Composition selon la revendication 4, **caractérisée en ce que** l'alpha-hydroxy acide est choisi parmi les acides glycolique, lactique, mandélique, citrique, thiolactique et thioglycolique.

6. Composition selon la revendication 1, **caractérisée en ce qu'**elle comprend en outre un mucopolysaccharide.

7. Utilisation d'un ester de diosgénine selon la revendication 1 pour la fabrication d'un médicament dermatologique agissant par action sur le système musculo-aponévrotique superficiel ou sur les récepteurs cellulaires des oestrogènes et de la progestérone, pour le traitement des affections de la peau.

8. Procédé de traitement cosmétique de la peau consistant à appliquer sur la zone à traiter, une quantité efficace d'ester de diosgénine selon la revendication 1.

## Claims

1. A composition which is useful in therapeutics and in cosmetology, for topical application, **characterized in that** it comprises a diosgenin ester comprising a linear or branched alkylated ester chain of 1 to 30 carbon atoms.

2. The composition of claim 1, **characterized in that** the diosgenin ester is selected from diosgenin lactate and diosgenin glycolate.

3. The composition of any one of claims 1 and 2, **characterized in that** the content of diosgenin ester is between 0.05% and 80% by weight relative to the total weight of the composition.

4. The composition of claim 1, **characterized in that** it also comprises an alpha-hydroxy acid or a salt of an alpha-hydroxy acid.

5. The composition of claim 4, **characterized in that** the alpha-hydroxy acid is selected from glycolic acid, lactic acid, mandelic acid, citric acid, thiolactic acid and thioglycolic acid.

6. The composition of claim 1, **characterized in that** it also comprises a mucopolysaccharide.

7. The use of a diosgenin ester as claimed in claim 1, for manufacturing a dermatological medicinal product acting by means of an action on the superficial musculoaponeurotic system or on the cellular estrogen and progesterone receptors, for the treatment of skin conditions.

8. A cosmetic treatment process for the skin, consisting in applying to the area to be treated an effective amount of diosgenin ester as claimed in claim 1.

## Patentansprüche

1. Zusammensetzung, die in den Bereichen der Therapeutik und der Kosmetik zur topischen Verabreichung geeignet ist, **dadurch gekennzeichnet, dass** sie einen Diosgeninester mit unverzweigter oder verzweigter alkylierter Esterkette mit 1 bis 30 Kohlenstoffatomen umfasst.

2. Zusammensetzung nach Anspruch 1, **dadurch gekennzeichnet, dass** der Diosgeninester aus Diosgeninlactat und -glykolat ausgewählt ist.

3. Zusammensetzung nach einem der Ansprüche 1 und 2, **dadurch gekennzeichnet, dass** der Gehalt an Diosgeninester zwischen 0,05 Gew.-% und 80 Gew.-%, bezogen auf das Gesamtgewicht der Zusammensetzung, beträgt.

4. Zusammensetzung nach Anspruch 1, **dadurch gekennzeichnet, dass** sie weiters eine α-Hydroxysäure oder ein Salz der α-Hydroxysäure umfasst.

5. Zusammensetzung nach Anspruch 4, **dadurch gekennzeichnet, dass** die α-Hydroxysäure aus Glykol-, Milch-, Mandel-, Zitronen-, Thiomilch- und Thioglykolsäure ausgewählt ist.

6. Zusammensetzung nach Anspruch 1, **dadurch gekennzeichnet, dass** sie weiters ein Mucopolysaccharid umfasst.

7. Verwendung eines Diosgeninesters nach Anspruch 1 zur Herstellung eines dermatologischen Medikaments, das durch Wirkung auf das oberflächliche Muskel-Faszien-System oder auf Zellrezeptoren von Östrogenen und von Progesteron funktioniert, zur Behandlung von Hauterkrankungen.

8. Kosmetisches Behandlungsverfahren der Haut, umfassend das Auftragen einer wirksamen Menge an Diosgeninester nach Anspruch 1 auf den zu behandelnden Bereich.
